# EUROPEAN PATENT SPECIFICATION

(11) **EP 2 376 153 B1**
(45) Date of publication and mention of the grant of the patent: **08.11.2017**
(21) Application number: 09775271.1
(22) Date of filing: 15.12.2009
(51) Int. Cl.: A61M 5/32, A61M 5/20

(54) **SAFETY SYRINGE FOR AUTOINJECTOR**
SICHERHEITSSPRITZE FÜR EIN AUTOMATISCHES INJEKTIONSGERÄT
SERINGUE DE SÉCURITÉ POUR AUTO-INJECTEUR

(30) Priority: 18.12.2008 GB 0823066
(43) Date of publication of application: 19.10.2011
(73) Proprietor: The Medical House Limited, Breakspear Way Hemel Hempstead Hertfordshire HP2 4UL (GB)
(72) Inventor: CLEATHERO, Ian Charles, Mowbray Leicestershire LE13 0FP (GB)
(74) Representative: HGF Limited
(86) International application number: PCT/GB2009/051716
(87) International publication number: WO 2010/070335

(56) References cited:
- EP-A1- 0 740 942
- EP-A1- 2 080 532
- EP-A2- 0 864 335
- WO-A1-2005/009520
- WO-A1-2010/026414
- US-A- 4 923 447
- US-A1- 2003 236 502
- US-A1- 2006 100 589
- US-B1- 6 203 530

## Description

This invention relates to a safety syringe, and more specifically to a safety syringe for incorporation into an autoinjector which is capable of shielding the needle after an injection has been delivered.

### BACKGROUND

Autoinjectors are well known in the art and provide automatic actuation means for delivering a dose of medicament to a patient. Many autoinjectors are designed around a syringe comprising a needle, barrel and plunger where the user actuates the device by pressing a button, for example, to actuate delivery. Autoinjectors offer an efficient and safe method for delivering an injection which is desirable for both inexperienced and regular users alike.

For autoinjectors to be economically appealing, it is preferable for them to be reusable and to be capable of using replaceable prefilled syringes. In prior art autoinjectors, to replace the syringe, the user must open the housing of the autoinjector, remove the used syringe and replace it with a new syringe. When removing the used syringe, however, the exposed needle presents a needle-stick injury risk to the user which is undesirable and, under some jurisdictions, unacceptable by law. It is therefore desirable for the needle to be protected and covered as the user removes the used syringe from the autoinjector.

Generally, the term 'safety syringe' is used to describe a syringe device that has means for protecting the user from accidental needle stick injury following the delivery of an injection. Additionally, safety syringes may be designed such that the syringe cannot be re-used. Many prior art safety syringes exist that are operated manually.

WO-A-2006/127484 describes a manual injection device that comprises a syringe and a needle guard. The needle guard is slidably arranged on the syringe and is biased from a first position wherein the needle is exposed toward a second position wherein the guard covers the needle. After an injection is manually delivered to a patient, the guard is locked in the second position to prevent the needle being exposed thereafter.

A safety syringe for use with an automatic injection system is described in WO-A-2005/009520. Again, the safety mechanism incorporates a needle shield that is extendable between a retracted position and an extended position that substantially covers the needle. The automatic injection system has a drive system that is configured to depress a plunger, thereby delivering a dose of medicament, and then move the shield to the extended position, upon activation. WO 2005/009520 thereby discloses all the features of the preamble of claim 1. In general, safety syringes are not identical in shape to standard prefilled syringes and include several additional features. Therefore, if a safety syringe is required to be sold as a prefilled unit, then modified filling equipment is needed which may lead to increased production costs. It is therefore an object of the present invention to alleviate the above-mentioned disadvantages.

### BRIEF SUMMARY OF THE DISCLOSURE

In accordance with a first aspect of the present invention there is provided a safety syringe with all the features of claim 1. Preferably, the safety syringe assembly comprises latching means that are capable of axially restraining said first sleeve relative to said syringe in said first and second positions and wherein, in said first position, said latching means are releasable.

In a preferred form, the safety syringe assembly includes a second sleeve that has a larger diameter than said first sleeve and is axially fixed relative to said syringe and wherein said latching means form part of said second sleeve.

According to a second aspect of the present invention there is provided a method of removing a safety syringe assembly from an autoinjector with all the features of claim 13. Further features of the invention are defined in the appended claims.

### BRIEF DESCRIPTION OF THE DRAWINGS

Embodiments of the invention are further described hereinafter with reference to the accompanying drawings, in which:
Figure 1 is a perspective view of a safety syringe assembly according to the present invention, with the needle shield removed;
Figure 2 is a perspective view of the safety syringe assembly of Figure 1, with the needle shield in place, but the outer cylinder removed; and
Figure 3 is a cross-sectional view of the outer cylinder.

### DETAILED DESCRIPTION

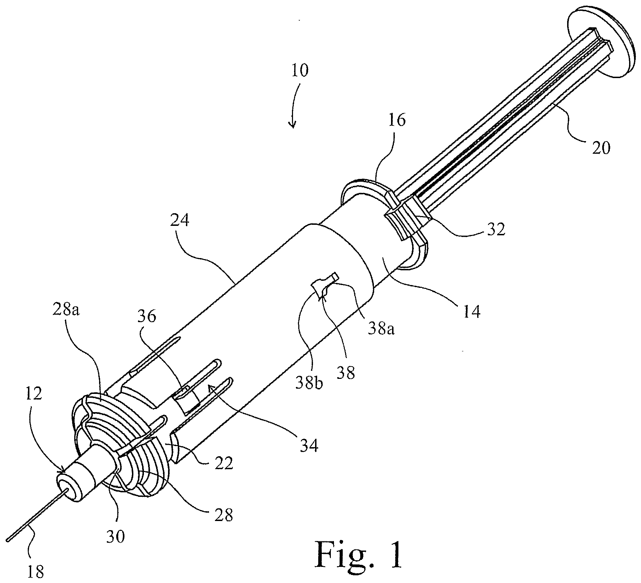

Figure 1 shows a safety syringe assembly 10 for use in an autoinjector (not shown). The safety syringe assembly 10 comprises a syringe 12, an inner sleeve 22 and an outer sleeve 24. In Figure 1, the outer sleeve 24 is shown to be transparent to improve clarity. The syringe has a barrel 14, a finger flange 16, a needle 18 and a plunger 20. The syringe 12 may be a "standard syringe" in accordance with a standard industry specification, or alternatively, the syringe may be any syringe 12 comprising the above mentioned components.

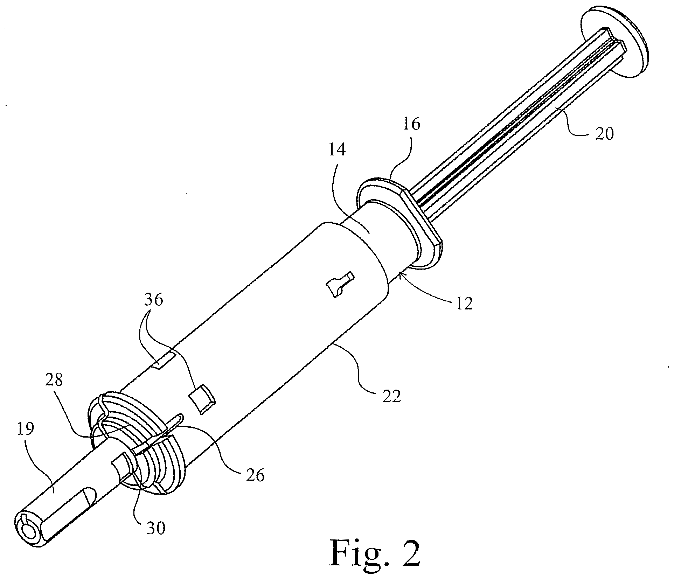

It is envisaged that the inner sleeve 22 and outer sleeve 24 can be fitted onto a prefilled syringe 12 prior to installing in an autoinjector. During assembly, the syringe 12 is inserted into the inner sleeve 22 so that the needle protrudes from an aperture 30 in a forward end. The inner sleeve 22 is shown more clearly in Figure 2 where it can be seen to comprise several axial slots 26 in a front portion 28. The front portion 28 has a radially protruding annular flange 28a that is discontinuous around its circumference due to the slots 26. The slots 26 lend the front portion 28 a small degree of radial flexibility that allows the syringe 12 to be installed in the inner sleeve 22. To do this, the front portion 28 must flex radially outwards to allow the needle shield 19 to pass through the aperture 30. This is because the needle shield 19 has a larger diameter than that of the aperture 30 when the front portion 28 is in a relaxed position. Once the needle shield 19 has passed through the aperture 30, the front portion 28 can return to its relaxed position and may move into a position intermediate a forward shoulder of the syringe barrel 14 and the needle shield 19. In the position shown in Figure 2, the syringe 12 is axially restrained from moving forwards by contact between the syringe barrel 14 and the front portion 28 of the inner cylinder 22. Since a force is required to remove the needle shield 19 from the needle 18, the syringe 12 is also axially restrained (for forces less than that required to remove the needle shield 19) from moving rearward, through contact between the needle shield 19 and the front portion 28 of the inner cylinder 22.

As shown in Figure 1, the outer cylinder 24 fits around the inner cylinder 22 and syringe 12. The outer cylinder 24 has rear clips 32 that clip onto the finger flange 16 of the syringe 12. The clip connection between the outer cylinder 24 and the syringe 12 is such that they are fixed, both axially and rotationally, with respect to one another. When installed over the inner cylinder 24, front legs 34 of the outer cylinder 24 clip into front windows 36 of the inner cylinder 22. The inner cylinder 22 and outer cylinder 24 may be supplied as a sub-assembly, with which the prefilled syringe 12 can be assembled at a later date. Therefore there is no disruption to the conventional syringe filling process; the safety features being fitted as a secondary stage once a sterile prefilled syringe has been created in accordance with the necessary regulations.

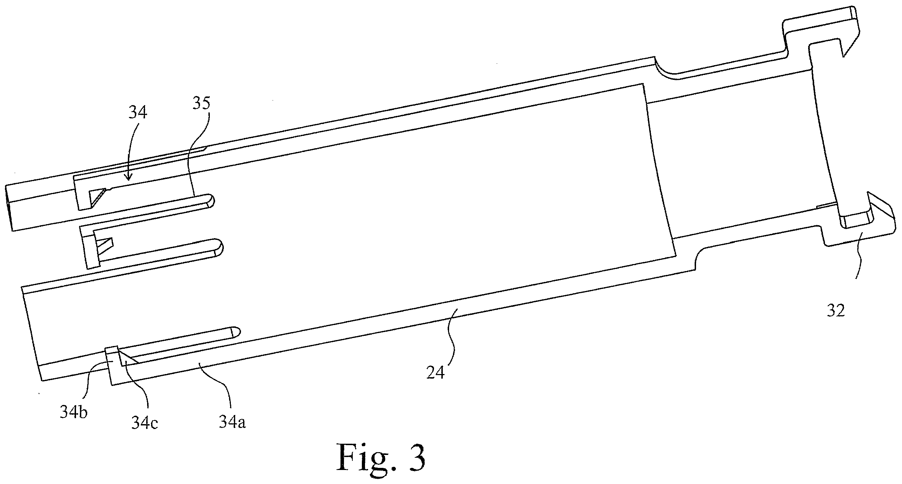

Figure 3 shows a cross sectional view of the outer cylinder 24 and shows the rear clips 32 and the front legs 34 in detail. As Figure 3 shows, the front legs 34 are defined by axial slots 35 in the surface of the outer cylinder 24. The front legs 34 comprise an axial leg element 34a and a radial leg element 34b (a "radially flexible tag") extending radially inwards therefrom. Intermediate the axial leg element 34a and the radial leg element 34b there is a ramp 34c, although it should be noted that the ramp does not extend across the entire width of the front legs 34. The front legs 34 are radially flexible allowing them to flex outwards when being installed onto the inner cylinder 24 and syringe 12. When installed, the front legs 34 are in a relaxed position with radial leg elements 34b disposed in the front windows 36 of the inner cylinder 22.

With the inner 22 and outer cylinders 24 installed onto the syringe 12, the safety syringe assembly 10 can be inserted into an autoinjector. It is envisaged that certain embodiments of the invention will be compatible with the autoinjector described in the applicant's PCT publication WO-A-2005/070481, however the present invention is not restricted to any one type of autoinjector. Nevertheless, it is preferred that the safety syringe assembly 10 be constrained by constraining means within the autoinjector through some contact between the autoinjector and the inner cylinder 22. In a preferable embodiment, it is contact between a part of the autoinjector and the annular flange 28a of the front portion 28 of the inner cylinder 22 that restrains the safety syringe assembly 10 within the autoinjector device.

With the safety syringe assembly installed in an autoinjector, the user actuates the autoinjector to deliver an injection. The needle is inserted into an injection site, either by manual or automatic means. Then, a drive mechanism provides a force to the plunger 20 to move it axially forwards within the syringe barrel 14, expelling medicament through the needle 18. When the delivery is complete, the plunger will be in a forward position within the syringe barrel 14 and the needle is withdrawn. Again, this may be a manual retraction or may occur automatically. For the autoinjector to deliver another injection, the used syringe 12 must be removed and replaced by another prefilled syringe. The present invention allows the removal and replacement of the entire safety syringe assembly 10.

It should be noted that since the claimed invention relates to a safety syringe assembly per se, it is not limited to any particular method or mechanism for drug delivery. The above described method merely serves as an illustrative example of how drug delivery might take place when the claimed apparatus is incorporated in an autoinjector device.

To remove the used safety syringe assembly 10 from the autoinjector, the user opens the autoinjector and applies a rearward axial force to the finger flange 16 of the syringe 12 and/or the outer cylinder 24. Since the safety syringe assembly 10 is otherwise axially restrained in the autoinjector, this causes the outer cylinder 24 and syringe 12 to move rearwardly with respect to the inner cylinder 22 and the autoinjector. In doing so, the front legs 34 of the outer cylinder 24 flex radially outwards as ramps 34c allow the radial leg elements 34b to ride out of the front windows 36. As the outer cylinder 24 and syringe move collectively rearwardly, the needle 18 is drawn into the inner cylinder 22 and is thereby entirely covered and protected.

The outer cylinder 24 and syringe 12 continue to move collectively rearwardly with respect to the inner cylinder 22 until the front legs 34 encounter rear windows located at a rear end on the inner cylinder 22. As shown in Figure 1, the rear windows 38 are in axial alignment with the front windows 36 and front legs 34 and are generally T-shaped, comprising an axial slot 38a and a circumferential slot 38b. When the front legs 34 encounter the rear windows 38, they flex radially inwards once more to a relaxed position with the radial leg elements 34b disposed in the circumferential slots 38b and the ramps 34c disposed within the axial slots 38a. Further rearward movement of the syringe 12 and outer cylinder 24 relative to the inner cylinder 22 is prevented by abutment between the radial leg elements 34b and the rear edges of circumferential slots 38b. The ramps 34c cannot assist the front legs 34 in riding out of the rear windows 38 since the ramps are disposed within the axial slots 38a that are sufficiently long that the ramps 34c do not contact the edge. Thus, when the front legs 34 relax radially into the rear windows 38, the outer cylinder 24 and syringe 12 are locked with respect to the inner cylinder 22.

As described above, at this moment, the needle 18 has been completely withdrawn into the inner cylinder 22 so that it is completely surrounded and protected. More importantly, however, the inner cylinder 22 provides a fixed shield around the needle 18 such that the needle is no longer exposed and there is no risk to the user of accidental needle stick injury when removing the safety syringe assembly 10 from the autoinjector.

As an alternative to the above described embodiment in which removal of the used syringe 12 from the autoinjector causes the needle to be shielded by the inner cylinder 22, the shielding could instead be deployed during (automatic or manual) retraction of the needle from the injection site after delivery of the medicament. In this latter embodiment, the needle would already be shielded by the inner cylinder 22 before the user opens the autoinjector to remove the used syringe.

With the needle within the inner cylinder 22, the safety syringe assembly 10 can be safely removed from the autoinjector. This may be done by operating release means on the autoinjector, or by simply pulling the safety syringe assembly 10 with sufficient force such that the restraining means holding the assembly 10 within the autoinjector are overcome.

The used safety syringe assembly 10 can then be safely disposed of in the usual way e.g. in a sharps bin. Since the safety syringe assembly 10 only consists of three components (i.e. the syringe 12, the inner cylinder 22 and the outer cylinder 24), each being relatively inexpensive, the assembly can be designed as a disposable unit. The simplicity of the assembly 10 also allows the inner 22 and outer 24 cylinders to be fitted onto prefilled standard syringes. Therefore, no new equipment or modification is necessary to fill the syringes.

In certain embodiments of the safety syringe assembly, it is preferable for the inner 22 and/or outer 24 cylinders to simultaneously operate as components of the autoinjector. For example, in some autoinjectors, the inner cylinder 22 could also serve as the syringe holder for axially restraining and translating the syringe within the autoinjector. This approach would lead to reduced autoinjector production costs since each autoinjector could be produced with fewer components.

It is clear from the above description that the present invention offers an effective and efficient apparatus and method for installing and removing a syringe assembly from an autoinjector whilst keeping the needle covered and the user protected. The apparatus comprises few components that may be made from relatively inexpensive materials and is simple in construction. Certain embodiments of the apparatus may also be made specifically for particular autoinjectors and may comprise components that also function as components of the autoinjector. For example, the inner cylinder 22 may comprise part or all of a syringe holder of the type described in our patent application WO 2007/083115.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example within the scope of the appended claims, described herein unless incompatible therewith.

All of the features disclosed in this specification (including any accompanying claims, abstract and drawings), and/or all of the steps of any method or process so disclosed, may be combined in any combination, except combinations where at least some of such features and/or steps are mutually exclusive within the scope of the appended claims. Each feature disclosed in this specification (including any accompanying claims, abstract and drawings), may be replaced by alternative features serving the same, equivalent or similar purpose within the scope of the appended claims, unless expressly stated otherwise. Thus, unless expressly stated otherwise, each feature disclosed is one example only of a generic series of equivalent or similar features.

The invention is not restricted to the details of any foregoing embodiment. The invention extends to any novel one, or any novel combination, of the features disclosed in this specification (including any accompanying claims, abstract and drawings), or to any novel one, or any novel combination, of the steps of any method or process so disclosed within the scope of the appended claims.

## Claims

1. A safety syringe assembly (10) adapted for use in an autoinjector comprising a syringe (12) having
a barrel (14) for holding a volume of medicament,
a needle (18) at one end of the barrel (14), and
a plunger (20), axially moveable within the barrel (14); and
a first sleeve (22) at least partially surrounding said syringe (12);
wherein said syringe (12) is axially moveable relative to said first sleeve (22) between a first position, in which said needle (18) is exposed, and a second position, in which said needle (18) is substantially surrounded by said first sleeve (22) and is not exposed; and
said syringe (12) is axially lockable with respect to said first sleeve (22) when in said second position;
**characterised in that** said first sleeve (22) comprises a radially flexible front portion (28), defined by a plurality of axial slots (26), where said radially flexible front portion (28) is capable of flexing radially outwards during assembly.

2. A safety syringe assembly (10) as claimed in claim 1, further comprising latching means that are capable of axially restraining said first sleeve (22) relative to said syringe (12) in said first and second positions and wherein, in said first position, said latching means are releasable.

3. A safety syringe assembly (10) as claimed in claim 2, further comprising a second sleeve (24) that has a larger diameter than said first sleeve (22) and is axially fixed relative to said syringe (12) and wherein said latching means form part of said second sleeve (24).

4. A safety syringe assembly (10) as claimed in claim 3, wherein said latching means includes one or more radially flexible tags (34b), which are preferably located at the end of a resiliently flexible leg (34).

5. A safety syringe assembly (10) as claimed in claim 4, wherein said first sleeve (22) includes a first set of one or more apertures (36) at a front end and a second set of one or more apertures (38) at a rear end, and wherein one or more of said flexible tags (34b) are moveable radially into and out of said apertures (36,38);
wherein, in said first position, one or more of said tags (34b) are engaged with said first set of one or more apertures (36), and in said second position, one or more of said tags (34b) are engaged with said second set of one or more apertures (38).

6. A safety syringe assembly (10) as claimed in claim 5, wherein one or more of said tags (34b) each comprises a ramp element (34c) that is capable of allowing said one or more tags (34b) to flex radially outwards, out of engagement with said first set of one or more apertures (36), permitting said first sleeve (22) to move from said first position to said second position, preferably wherein said second set of one or more apertures (38) is shaped such that in said second position said ramp elements (34c) prevent said one or more tags (34b) flexing radially outwards, out of engagement with said second set of one or more apertures (38).

7. A safety syringe assembly (10) as claimed in claim 6, wherein said second set of one or more apertures (38) is T-shaped comprising an axial slot (38a) and a circumferential slot (38b); and
in said second position, said ramp elements (34c) are disposed within said axial slots (38a) of said second set of one or more apertures (38) and are not in contact with said first sleeve (22); and
said second sleeve (24) is axially lockable relative said first sleeve (22) due to abutment between said one or more tags (34b) and edges of said circumferential slots (38b).

8. A safety syringe assembly (10) as claimed in any of claims 3-7, wherein said barrel (14) further comprises a finger flange (16) and said second sleeve (24) is axially fixed to said finger flange by clips (32).

9. A safety syringe assembly (10) as claimed in any preceding claim, wherein said first sleeve (22) is a component of an autoinjector and acts as a syringe support means supporting the barrel (14) at an axial location at or forward of the forwardmost position of the plunger (20) and having a reaction surface for the syringe (12), whereby said reaction surface is configured to provide an axial compressive force on said barrel (14) when a forward axial force is applied to the plunger (20).

10. A safety syringe assembly (10) as claimed in any preceding claim, wherein said syringe (12) is axially moveable relative to said first sleeve (22) from said first position to said second position in response to removal of the safety syringe assembly (10) from an autoinjector.

11. A safety syringe assembly (10) as claimed in any of claims 1-9, wherein said syringe (12) is axially moveable relative to said first sleeve (22) from said first position to said second position in response to retraction of the needle (18) from an injection site after delivery of medicament.

12. An autoinjector comprising a safety syringe assembly (10) as claimed in any of claims 3-9, wherein said first sleeve (22) is axially restrained within said autoinjector and said first sleeve (22) is configured to be moveable from said first position to said second position by rearward axial movement of said syringe (12) and second sleeve (24) relative to said first sleeve (22).

13. A method of removing a safety syringe assembly (10) from an autoinjector wherein said safety syringe assembly (10) comprises
a syringe (12) having
a barrel (14) for holding a volume of medicament,
a needle (18) at one end of the barrel (14), and
a plunger (20), axially moveable within the barrel (14); and
a first sleeve (22) that surrounds said syringe (12) and is axially restrained by restraining means within said autoinjector device;
wherein said syringe (12) is axially moveable relative to said first sleeve (22) between a first position, in which said needle (18) is exposed, and a second position, in which said needle (18) is surrounded by said first sleeve (22) and is not exposed; and
said syringe (12) is locked with respect to said first sleeve (22) when in said second position;
said method comprising the steps of
a) opening said autoinjector to provide access to said safety syringe assembly (10);
b) axially moving said syringe (12) relative to said first sleeve (22) from said first position in which said needle (18) is exposed to said second position in which said syringe (12) is locked with respect to said first sleeve (22) and said needle (18) is not exposed;
c) exerting a rearward axial force to said syringe (12) and removing said syringe (12) and said first sleeve (22) from said autoinjector wherein said rearward axial force is sufficient to overcome said restraining means;
wherein step c) is executed after steps a) and b) have been completed in any order.

14. The method of claim 13 in which step b) occurs prior to step a) and occurs during automatic or manual retraction of said needle (18) from an injection site following delivery of a medicament.

15. A method of removing a safety syringe assembly (10) from an autoinjector as claimed in claim 13 or 14, wherein said safety syringe assembly (10) further comprises any of the features of claims 2-11.

## Patentansprüche

1. Sicherheitsspritzenbaugruppe (10), geeignet zur Verwendung in einem Autoinjektionsgerät, aufweisend
eine Spritze (12) mit
einem Zylinder (14) zum Fassen eines Volumens an Medikament,
eine Nadel (18) an einem Ende des Zylinders (14) und
einen Kolben (20), der axial innerhalb des Zylinders (14) bewegbar ist, und
eine die Spritze (12) mindestens teilweise umgebende erste Hülse (22),
wobei die Spritze (12) axial relativ zur ersten Hülse (22) zwischen einer ersten Position, in welcher die Nadel (18) freigelegt ist, und einer zweiten Position, in welcher die Nadel (18) im Wesentlichen von der ersten Hülse (22) umgeben ist und nicht freigelegt ist, bewegbar ist, und
die Spritze (12) in Bezug auf die erste Hülse (22) axial verriegelbar ist, wenn sie in der zweiten Position ist,
**dadurch gekennzeichnet, dass** die erste Hülse (22) einen radial flexiblen vorderen Abschnitt (28) aufweist, der durch eine Vielzahl von Axialschlitzen (26) definiert ist, wobei der radiale flexible vordere Abschnitt (28) in der Lage ist, sich während des Zusammensetzens radial nach außen zu biegen.

2. Sicherheitsspritzenbaugruppe (10) nach Anspruch 1, ferner aufweisend Einrastmittel, die in der Lage sind, die erste Hülse (22) relativ zur Spritze (12) in der ersten und der zweiten Position axial zurückzuhalten, und wobei, in der ersten Position, die Einrastmittel lösbar sind.

3. Sicherheitsspritzenbaugruppe (10) nach Anspruch 2, ferner aufweisend eine zweite Hülse (24), die einen größeren Durchmesser als die erste Hülse (22) hat und relativ zur Spritze (12) axial befestigt ist, und wobei die Einrastmittel Teil der zweiten Hülse (24) sind.

4. Sicherheitsspritzenbaugruppe (10) nach Anspruch 3, wobei das Einrastmittel eine oder mehrere radial flexible Laschen (34b) umfasst, welche sich vorzugsweise am Ende eines federnd flexiblen Fuß (34) befinden.

5. Sicherheitsspritzenbaugruppe (10) nach Anspruch 4, wobei die erste Hülse (22) einen ersten Satz einer oder mehrerer Öffnungen (36) an einem vorderen Ende und einen zweiten Satz einer oder mehrerer Öffnungen (38) an einem hinteren Ende umfasst, und wobei eine oder mehrere der flexiblen Laschen (34b) radial in die und aus den Öffnungen (36,38) bewegbar sind,
wobei in der ersten Position eine oder mehrere der Laschen (34b) in Eingriff mit dem ersten Satz einer oder mehrerer Öffnungen (36) sind und in der zweiten Position eine oder mehrere der Laschen (34b) in Eingriff mit dem zweiten Satz einer oder mehrerer Öffnungen (38) sind.

6. Sicherheitsspritzenbaugruppe (10) nach Anspruch 5, wobei eine oder mehrere der Laschen (34b) jeweils ein Rampenelement (34c) aufweisen, das in der Lage ist, zu ermöglichen, dass die eine oder mehreren Laschen (34b) sich radial nach außen außer Eingriff mit dem ersten Satz einer oder mehrerer Öffnungen (36) biegen, was erlaubt, dass die erste Hülse (22) sich von der ersten Position zur zweiten Position bewegt, vorzugsweise wobei der zweite Satz einer oder mehrerer Öffnungen (38) so geformt ist, dass in der zweiten Position die Rampenelemente (34c) verhindern, dass die eine oder mehreren Laschen (34b) sich radial nach außen außer Eingriff mit dem zweiten Satz einer oder mehrerer Öffnungen (38) biegen.

7. Sicherheitsspritzenbaugruppe (10) nach Anspruch 6, wobei der zweite Satz einer oder mehrerer Öffnungen (38) T-förmig, aufweisend einen Axialschlitz (38a) und einen Umfangsschlitz (38b) ist und
in der zweiten Position die Rampenelemente (34c) innerhalb der Axialschlitze (38a) des zweiten Satzes einer oder mehrerer Öffnungen (38) angeordnet und nicht in Berührung mit der ersten Hülse (22) sind und
die zweite Hülse (24) axial relativ zur ersten Hülse (22) aufgrund von Anschlag zwischen der einen oder mehreren Laschen (34b) und Ränder der Umfangsschlitze (38b) verriegelbar ist.

8. Sicherheitsspritzenbaugruppe (10) nach einem der Ansprüche 3-7, wobei der Zylinder (14) ferner einen Fingerflansch (16) aufweist und die zweite Hülse (24) axial am Fingerflansch durch Haltevorrichtungen (32) befestigt ist.

9. Sicherheitsspritzenbaugruppe (10) nach einem der vorhergehenden Ansprüche, wobei die erste Hülse (22) eine Komponente eines Autoinjektionsgeräts ist und als ein Spritzenunterstützungsmittel wirkt, das den Zylinder (14) an einer axialen Stelle an oder vor der vordersten Position des Kolbens (20) unterstützt und eine Reaktionsfläche für die Spritze (12) hat, wobei diese Reaktionsfläche gestaltet ist, um einer axiale Druckkraft auf dem Zylinder (14) bereitzustellen, wenn eine nach vorne gerichtete axiale Kraft auf den Kolben (20) aufgebracht wird.

10. Sicherheitsspritzenbaugruppe (10) nach einem der vorhergehenden Ansprüche, wobei die Spritze (12) axial relativ zur ersten Hülse (22) von der ersten Position zur zweiten Position als Reaktion auf das Entfernen der Sicherheitspritzenbaugruppe (10) aus dem Autoinjektionsgerät bewegbar ist.

11. Sicherheitsspritzenbaugruppe (10) nach einem der Ansprüche 1-9, wobei die Spritze (12) axial relativ zur ersten Hülse (22) von der ersten Position zur zweiten Position als Reaktion auf das Einziehen der Nadel (18) aus einer Injektionsstelle nach Abgabe eines Medikaments bewegbar ist.

12. Autoinjektionsgerät, aufweisend eine Sicherheitsspritzenbaugruppe (10) nach einem der Ansprüche 3-9, wobei die erste Hülse (22) axial innerhalb des Autoinjektionsgeräts zurückgehalten wird und die erste Hülse (22) gestaltet ist, um von der ersten Position zur zweiten Position bewegbar zu sein durch rückwärtige axiale Bewegung der Spritze (12) und zweiten Hülse (24) relativ zur ersten Hülse (22).

13. Verfahren zum Entfernen einer Sicherherheitsspritzenbaugruppe (10) aus einem Autoinjektionsgerät, wobei die Sicherherheitsspritzenbaugruppe (10) Folgendes aufweist
eine Spritze (12) mit
einem Zylinder (14) zum Fassen eines Volumens an Medikament,
eine Nadel (18) an einem Ende des Zylinders (14) und
einen Kolben (20), der axial innerhalb des Zylinders (14) bewegbar ist, und
eine erste Hülse (22), die die Spritze (12) umgibt und axial durch Rückhaltemittel innerhalb der Autoinjektionsvorrichtung zurückgehalten wird,
wobei die Spritze (12) axial relativ zur ersten Hülse (22) zwischen einer ersten Position, in welcher die Nadel (18) freigelegt ist, und einer zweiten Position, in welcher die Nadel (18) von der ersten Hülse (22) umgeben ist und nicht freigelegt ist, bewegbar ist und
die Spritze (12) in Bezug auf die erste Hülse (22) verriegelt ist, wenn sie in der zweiten Position ist,
**dadurch gekennzeichnet, dass**
die erste Hülse (22) einen radial flexiblen vorderen Abschnitt (28) aufweist, der durch eine Vielzahl von Axialschlitzen (26) definiert ist, wobei der radiale flexible vordere Abschnitt (28) in der Lage ist, sich während des Zusammensetzens radial nach außen zu biegen,
wobei das Verfahren folgende Schritte umfasst
a) Öffnen des Autoinjektionsgeräts, um Zugang zur Sicherherheitsspritzenbaugruppe (10) zu bieten;
b) axiales Bewegen der Spritze (12) axial relativ zur ersten Hülse (22) von der ersten Position, in welcher die Nadel (18) freigelegt ist, zur zweiten Position, in welcher die Spritze (12) in Bezug auf die erste Hülse (22) verriegelt ist und die Nadel (18) nicht freigelegt ist,
c) Ausüben einer rückwärtigen axialen Kraft auf die Spritze (12) und Entfernen der Spritze (12) und der ersten Hülse (22) aus dem Autoinjektionsgerät, wobei die rückwärtige axiale Kraft ausreichend ist, um die Rückhaltemittel zu überwinden,
wobei Schritt c) nachdem die Schritte a) und b) in jeglicher Reihenfolge abgeschlossen worden sind.

14. Verfahren nach Anspruch 13, in welchem Schritt b) vor Schritt a) stattfindet und während dem automatischen oder manuellen Einziehen der Nadel (18) aus einer Injektionsstelle nach Abgabe eines Medikaments stattfindet.

15. Verfahren zum Entfernen einer Sicherheitsspritzenbaugruppe (10) aus einem Autoinjektionsgerät nach Anspruch 13 oder 14, wobei die Sicherheitsspritzenbaugruppe (10) ferner die Ausrüstungen nach Anspruch 2-11 aufweist.

## Revendications

1. Ensemble de seringue de sécurité (10) adapté pour être utilisé dans un auto-injecteur, comprenant
une seringue (12), possédant
un corps (14) pouvant contenir un volume de médicament,
une aiguille (18) à un bout du corps (14), et
un piston (20) pouvant être déplacé axialement au sein du corps (14) ; et
un premier manchon (22) entourant, au moins partiellement, ladite seringue (12) ;
ladite seringue (12) pouvant être déplacée axialement relativement audit premier manchon (22) entre une première position, dans laquelle ladite aiguille (18) est exposée, et une deuxième position, dans laquelle ladite aiguille (18) est substantiellement entourée par ledit premier manchon (22) et n'est pas exposée ; et
ladite seringue (12) étant axialement verrouillable relativement audit premier manchon (22) lorsqu'elle se trouve dans ladite deuxième position;
**caractérisée en ce que** ledit premier manchon (22) comprend une partie antérieure radialement flexible (28) définie par une pluralité de fentes axiales (26), ladite partie antérieure radialement flexible (28) étant en mesure de fléchir radialement vers l'extérieur au cours de l'assemblage.

2. Ensemble de seringue de sécurité (10) selon la revendication 1, comprenant en outre un dispositif de verrouillage capable de retenir axialement ledit premier manchon (22) relativement à ladite seringue (12) dans lesdites première et deuxième positions, et dans lequel, dans ladite première position, ledit dispositif de verrouillage est détachable.

3. Ensemble de seringue de sécurité (10) selon la revendication 2, comprenant en outre un deuxième manchon (24) dont le diamètre est supérieur à celui dudit premier manchon (22) et axialement fixe relativement à ladite seringue (12), et ledit dispositif de verrouillage faisant partie dudit deuxième manchon (24).

4. Ensemble de seringue de sécurité (10) selon la revendication 3, ledit dispositif de verrouillage comprenant une ou plusieurs pattes radialement flexibles (34b), situées de préférence au bout d'un montant souple et élastique (34).

5. Ensemble de seringue de sécurité (10) selon la revendication 4, ledit premier manchon (22) comprenant un premier ensemble d'une ou plusieurs ouvertures (36) à une extrémité antérieure et un deuxième ensemble d'une ou plusieurs ouvertures (38) à une extrémité postérieure, et une ou plusieurs desdites pattes flexibles (34b) pouvant être déplacées radialement dans et hors desdites ouvertures (36,38) ;
dans laquelle, dans ladite première position, une ou plusieurs desdites pattes (34b) s'engagent avec ledit premier ensemble d'une ou plusieurs ouvertures (36), et dans ladite deuxième position, une ou plusieurs desdites pattes (34b) s'engagent avec ledit deuxième ensemble d'une ou plusieurs ouvertures (38).

6. Ensemble de seringue de sécurité (10) selon la revendication 5, une ou plusieurs desdites pattes (34b) comprenant chacune un élément de rampe (34c) capable de permettre le fléchissement vers l'extérieur desdites une ou plusieurs pattes (34b), hors de contact avec ledit premier ensemble d'une ou plusieurs ouvertures (36), en permettant le déplacement dudit manchon (22) de ladite première position à ladite deuxième position, de préférence ledit deuxième ensemble d'une ou plusieurs ouvertures (38) étant façonné de sorte que dans ladite deuxième position lesdits éléments de rampe (34c) empêchent lesdites une ou plusieurs pattes (34b) de fléchir radialement vers l'extérieur, en se dégageant dudit deuxième ensemble d'une ou plusieurs ouvertures (38).

7. Ensemble de seringue de sécurité (10) selon la revendication 6, ledit deuxième ensemble d'une ou plusieurs ouvertures (38) étant façonné en forme de « T », comprenant une fente axiale (38a) et une fente circonférentielle (38b) ; et
dans ladite deuxième position, lesdits éléments de rampe (34c) sont disposés au sein desdites fentes axiales (38a) dudit deuxième ensemble d'une ou plusieurs ouvertures (38), et ne sont pas en contact avec ledit premier manchon (22) ; et
ledit deuxième manchon (24) pouvant être verrouillé axialement relativement audit premier manchon (22) en raison de la butée entre lesdites une ou plusieurs pattes (34b) et les bords desdites fentes circonférentielles (38b).

8. Ensemble de seringue de sécurité (10) selon une quelconque des revendications 3 à 7, ledit corps (14) comprenant en outre une collerette à doigt (16) et ledit deuxième manchon (24) étant fixé axialement sur ladite collerette à doigt par des clips (32).

9. Ensemble de seringue de sécurité (10) selon une quelconque des revendications précédentes, ledit premier manchon (22) étant un composant d'un auto-injecteur, et jouant un rôle de support de seringue soutenant le corps (14) en un emplacement axial dans la position la plus avancée du piston (20), ou une position antérieure à celle-ci, et présentant une surface de réaction pour la seringue (12), ladite surface de réaction étant configurée pour appliquer une force de compression axiale sur ledit corps (14) lors de l'application d'une force axiale vers l'avant sur le piston (20).

10. Ensemble de seringue de sécurité (10) selon une quelconque des revendications précédentes, ladite seringue (12) pouvant être déplacée axialement relativement audit premier manchon (22) de ladite première position à ladite deuxième position en réponse à l'enlèvement de l'ensemble de seringue de sécurité (10) d'un auto-injecteur.

11. Ensemble de seringue de sécurité (10) selon une quelconque des revendications 1 à 9, ladite seringue (12) pouvant être déplacée axialement relativement audit premier manchon (22) de ladite première position à ladite deuxième position en réponse à la rétraction de l'aiguille (18) d'un lieu d'une injection après l'administration du médicament.

12. Auto-injecteur comprenant un ensemble de seringue de sécurité (10) selon une quelconque des revendications 3 à 9, ledit premier manchon (22) étant retenu axialement au sein dudit auto-injecteur et ledit premier manchon (22) étant configuré pour être déplacé de ladite première position à ladite deuxième position sous l'effet du déplacement axial vers l'arrière de ladite seringue (12) et du deuxième manchon (24) relativement audit premier manchon (22).

13. Méthode d'enlèvement d'un ensemble de seringue de sécurité (10) d'un auto-injecteur, ledit ensemble de seringue de sécurité (10) comprenant :
une seringue (12) comprenant
un corps (14) pouvant contenir un volume de médicament,
une aiguille (18) à un bout du corps (14), et
un piston (20) pouvant être déplacé axialement au sein du corps (14) ; et
un premier manchon (22) entourant ladite seringue (12), et étant retenu axialement par un dispositif de retenue au sein dudit dispositif auto-injecteur ;
ladite seringue (12) pouvant être déplacée axialement relativement audit premier manchon (22) entre une première position, dans laquelle ladite aiguille (18) est exposée, et une deuxième position, dans laquelle ladite aiguille (18) est entourée par ledit premier manchon (22), et n'est pas exposée ; et ladite aiguille (12) étant bloquée relativement au premier manchon (22) lorsqu'elle se trouve dans ladite deuxième position ;
**caractérisée en ce que**
ledit premier manchon (22) comprend une partie antérieure radialement flexible (28) définie par une pluralité de fentes axiales (26), ladite partie antérieure radialement flexible (28) étant en mesure de fléchir radialement vers l'extérieur au cours de l'assemblage ;
ladite méthode comprenant les étapes
a) d'ouverture dudit auto-injecteur pour permettre l'accès audit ensemble de seringue de sécurité (10) ;
b) de déplacement axial de ladite seringue (12) relativement audit premier manchon (22) de la première position, dans laquelle ladite aiguille (18) est exposée à ladite deuxième position, dans laquelle ladite seringue (12) est bloquée relativement audit premier manchon (22), et ladite aiguille (18) n'est pas exposée ;
c) d'application sur ladite seringue (12) d'une force axiale vers l'arrière, et l'enlèvement de ladite seringue (12) et dudit premier manchon (22) dudit auto-injecteur, ladite force axiale vers l'arrière étant suffisante pour surmonter ledit dispositif de retenue ;
l'étape c) étant exécutée après l'exécution des étapes a) et b) dans un ordre quelconque.

14. Méthode selon la revendication 13, dans laquelle l'étape b) a lieu préalablement à l'étape a), et au cours de la rétraction automatique ou manuelle de ladite aiguille (18) du lieu d'une injection, à la suite de l'administration d'un médicament.

15. Méthode d'enlèvement d'un ensemble de seringue de sécurité (10) d'un auto-injecteur, selon la revendication 13 ou 14, ladite seringue de sécurité (10) comprenant en outre une quelconque des caractéristiques des revendications 2 à 11.
